(19) Europäisches Patentamt
European Patent Office
Office européen des brevets

(11) EP 0 865 765 B1

(12) # EUROPÄISCHE PATENTSCHRIFT

(45) Veröffentlichungstag und Bekanntmachung des Hinweises auf die Patenterteilung:
**14.07.2004 Patentblatt 2004/29**

(51) Int Cl.[7]: **A61B 8/13**, A61B 5/0456, G01S 7/52, G01S 15/89

(21) Anmeldenummer: **98105003.2**

(22) Anmeldetag: **19.03.1998**

(54) **Verfahren und Vorrichtung zur Erfassung von diagnostisch verwertbaren, dreidimensionalen Ultraschallbilddatensätzen**

Method and device for detection of diagnostically usable three dimensional ultrasonic picture data sets

Procédé et dispositif pour détecter des signaux ultrasoniques en trois dimensions qui sont utilisable en diagnostique

(84) Benannte Vertragsstaaten:
**AT BE CH DE DK ES FI FR GB GR IE IT LI LU NL PT SE**

(30) Priorität: **22.03.1997 DE 19712107**

(43) Veröffentlichungstag der Anmeldung:
**23.09.1998 Patentblatt 1998/39**

(73) Patentinhaber: **Echotech 3D Imaging Systems GmbH**
**85399 Hallbergmoos (DE)**

(72) Erfinder: **Polz, Hans, Dr.**
**85456 Wartenberg (DE)**

(74) Vertreter: **Graf, Helmut, Dipl.-Ing. et al**
**Patentanwälte Wasmeier & Graf**
**Postfach 10 08 26**
**93008 Regensburg (DE)**

(56) Entgegenhaltungen:
**EP-A- 0 668 051**
**EP-A- 0 736 284**
**WO-A-91/03792**
**DE-A- 19 541 987**
**US-A- 5 329 929**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 865 765 B1

Printed by Jouve, 75001 PARIS (FR)

## Beschreibung

Die Erfindung bezieht sich auf ein Verfahren gemäß Oberbegriff Patentanspruch 1 sowie auf eine Vorrichtung zum Durchführen dieses Verfahrens gemäß Oberbegriff Patentanspruch 6.

Ein derartiges Verfahren bzw. eine derartige Vorrichtung sind bekannt (EP-A-0 736 284). Bei diesem Verfahren wird unter Verwendung eines Ultraschallgerätes mit frei geführtem Ultraschallkopf eine Vielzahl von Ultraschalleinzelbilder eines zu untersuchenden Volumens gebildet und unter Verwendung eines Positions-Sensorsystems die Position des Ultraschallkopfes und damit die räumliche Lage der Bildebenen der generierten Ultraschallbilder bestimmt. Aus den Bilddaten der Einzelbilder und den Positions- und Orientierungsdaten wird dann ein dreidimensionaler, das zu untersuchende Volumen tomographisch erfassender Datensatz generiert, der aus einer Vielzahl von Voxeln mit jeweils einem zugeordneten Bild- oder Grauwert besteht und in dem die Lage der Voxel jeweils auf ein gemeinsames kartesisches Koordinatensystem bezogen ist. Für jedes Voxel wird der zugehörige Bild- oder Grauwert nach einem vorgegebenen Algorithmus gebildet, und zwar zur Ausfüllung von Lücken durch Interpolation, die bei der Bilderfassung aufgetreten sind.

Bekannt ist weiterhin ein Verfahren (US 41 00 916), bei dem einzelne Ultraschallbilder, die von einem mit Ultraschall arbeitenden Sensorsystem ermittelt werden, unter Berücksichtigung von Position- und Orientierungsdaten hinsichtlich ihrer Lage im Raum definiert werden. Ein dreidimensionaler diagnostisch verwertbarer Datensatz, der eine tomographische Bilderfassung von einem zu untersuchenden Volumen ermöglicht, wird allerdings nicht generiert.

Der Erfindung liegt die Aufgabe zugrunde, eine Vorrichtung aufzuzeigen, mit der bei freier manueller Führung des Ultraschallwandlers eine tomographische Erfassung eines gesamten, zu untersuchenden Volumens oder dreidimensionalen Raumes durch einen vollständigen dreidimensionalen Datensatz möglich ist.

Zur Lösung dieser Aufgabe ist ein Verfahren entsprechend dem kennzeichnenden Teil des Patentanspruches 1 ausgeführt und eine Vorrichtung entsprechend dem kennzeichnenden Teil des Patentanspruches 6 ausgebildet.

Auch bei der Erfindung wird bei freier Führung des Ultraschallwandlers entlang eines zu untersuchenden Volumens für eine tomographische Bilderfassung ein dreidimensionaler Datensatz generiert, der der tomographischen Bilderfassung entsprechend aus einer Vielzahl von Einzelbildern besteht, die das gesamte, zu untersuchende Volumen abdecken und sich ihrerseits aus einer Vielzahl von Bildwerten zusammensetzen. Durch die erfindungsgemäße Art der Bildung der einzelnen Bildwerte (Voxel) des dreidimensionalen Datensatzes werden bei der Bilderfassung aufgetretene Lücken ausgefüllt, so daß ein vollständiger dreidimensionaler Datensatz erhalten wird.

Die Erfindung wird im folgenden anhand der Figuren an einem Ausführungsbeispiel näher erläutert. Es zeigen:

- Fig. 1 in schematischer Darstellung eine Ausführungsform der erfindungsgemäßen Vorrichtung zur Generierung von diagnostisch verwertbaren dreidimensionalen Datensätzen für eine tomografische Bilderfassung eines zu untersuchenden Volumens;
- Fig. 2 in schematischer Darstellung die jeweils im Bildverarbeitungssystem abgelegten Einzelbilder, zusammen mit graphischen Darstellungen des EKG-Zyklus und des Respirations-Zyklus eines Patienten;
- Fig. 3 - 5 ein Ablaufdiagramm der Arbeitsweise der erfindungsgemäßen Vorrichtung;
- Fig. 6 eine schematische Darstellung über die Transformation der Rohdaten in einen verwertbaren dreidimensionalen Datensatz;
- Fig. 7 eine schematische Darstellung zur Erläuterung der Transformation der Rohdaten in den auf ein gemeinsames Bezugssystem bezogenen Datensatz.

In der Fig. 1 ist 1 ein Bildverarbeitungssystem, welches im wesentlichen aus dem Rechner 2 und aus den an diesen angeschlossenen peripheren Geräten, wie Tastatur 3, Bildschirm 4 und Haussteuerung 5 usw. besteht.

Diesem Bildverarbeitungssystem sind zugeordnet:

- Ein Ultraschallgerät 6 mit Ultraschall-Kopf 7;
- ein elektromagnetisches Lage- bzw. Positionserfassungsgerät oder ein elektromagnetischer Sensor 8 mit einem Sender 9 und einem von Magnetspulen gebildeten Empfänger 10;
- ein EKG-Gerät 11 mit zugehörigen Elektroden 12;
- ein Gerät 13 zur Registrierung der Atemlage (Respirationszyklus) mit zugehörigen Sonden 14.

Mit 15 ist ein Patient bezeichnet, dessen Körper oder Körperbereich ein zu untersuchendes Volumen bildet. Der Patient 15 ist auf einer Liege 16 angeordnet.

Bei dem Ultraschallgerät 6 mit dem Ultraschallkopf 7 handelt es sich um ein dem Fachmann bekanntes und in der ärztlichen Diagnostik verwendetes Gerät zur Erzeugung von Ultraschall-Bildern, die jeweils einen Bereich des zu untersuchenden Volumens wiedergeben, der sich in der Bildebene des freigeführten Ultraschallkopfes 7 befindet. Am Ultraschallkopf 7, der von der untersuchenden Persan (Arzt) manuell freigeführt wird, ist vorzugsweise ein Halter 17 vorgesehen, an welchem sich auch der Empfänger 10 des Sensorsystems 8 befindet.

Das System liefert aufgrund der von dem Sender 9 abgestrahlten Magnetfelder, die von dem Empfänger 10 bzw. den dortigen Spulen erfaßt werden, an seinem Ausgang Sensor-Daten (Positions- und Rotationsrohdaten), mit denen die Position und Orientierung des Empfängers 10 im Raum genau definiert ist, und zwar durch Translationsdaten in der X-, Y- und Z-Achse sowie durch Rotationsdaten um diese Achsen.

Ein solches elektromagnetisches Sensorsystem ist dem Fachmann bekannt, und zwar beispielsweise als ISOTRAK II der Firma POLHEMUS, One Hercules Drive, P.O.Box 560, Colchester, VT 05446.

Das EKG-Gerät 11 ist ein solches, welches dem Fachmann aus der ärztlichen Praxis bzw. Diagnostik bekannt ist und welches in Abhängigkeit von dem EKG-Zyklus an einem Ausgang eine sich ändernde Spannung liefert, die im Diagramm a der Fig. 2 wiedergegeben ist und die typischen positiven und negativen R-Spitzen aufweist, zwischen denen jeweils ein R-Intervall gebildet ist. Bei dem Gerät 13 handelt es sich ebenfalls um ein dem Fachmann bekanntes Gerät, welches in der ärztlichen Praxis bzw. Diagnostik zur Erfassung des Respirationszyklus verwendet wird und in Abhängigkeit von diesem Repirationszyklus am Ausgang eine sich ändernde Spannung liefert, die im Diagramm b der Fig. 2 wiedergegeben ist.

Wie in der Fig. 2 auch wiedergegeben ist, wird im Bildverarbeitungssystem 1 in digitaler Form bei jedem Einzelbild zusätzlich zu dem Bildinhalt auch ein zugeordneter Bildheader abgelegt, der zusätzliche Informationen enthält, beispielsweise allgemeine Informationen zum Bild, einen Meßwert, der die jeweilige Phasenlage des Bildes im EKG-Zyklus bestimmt, einen Meßwert der die Phasenlage des jeweiligen Bildes in dem Respirationszyklus bestimmt sowie die Positions- und Orientierungsdaten, die die Lage der Bildebene des jeweiligen Einzelbildes im Raum bestimmen.

Um eine definierte Lage- bzw. Positionsbeziehung der jeweiligen Bildebene zu erreichen, wird das elektromagnetische Sensorsystem 8 verwendet, dessen Empfänger 10, der wenigstens eine Empfängerspule aufweist, an dem Halter 17 des freigeführten Schallwandlers oder Ultraschallkopfes 7 vorgesehen ist, und zwar in einer vorgegebenen räumlichen Beziehung aktiven Teil bzw. der Bildebene dieses Schallwandler.

Bei der Generierung der Positions- und Orientierungs-Daten (Translationdaten für X-, Y- und Z-Achse sowie Rotationdaten um diese Achsen) wird dann die räumliche Beziehung zwischen der Bildebene des Ultraschallkopfes 7 und dem Empfänger des Sensorsystems durch Kalibrierung, d.h. unter Verwendung von speziellen Kalibrier-Parametern errechnet. Hiermit ist es dann u.a. auch möglich, den Halter 17 mit dem Empfänger 10 an unterschiedlichen Ultraschall-Köpfen zu verwenden und diese jeweils durch unterschiedliche Kalibrierung oder Umrechnung der von dem Empfänger des Sensorsystems gelieferten Roh-Positionsdaten zu berücksichtigen.

Die Kalibrierung berücksichtigt somit die geometrische Lagebeziehung zwischen dem Empfänger und der Orientierung des aktiven Teils (Kristall) des Ultraschallkopfes 7 bzw. der Ebene (Bildebene), in der das vom Ultraschallkopf 7 erzeugte Bild liegt.

Bei der Erfassung der Bilder kommt es nicht auf die tatsächliche Lage der jeweiligen Bildebene im Raum an, sondern für die tomografische Bilderfassung reicht es aus, die relative Lage der einzelnen Bildebenen zueinander zu erfassen. Der Sender 9 des Sensorsystems 8 kann also im Umfeld der Vorrichtung beliebig positioniert werden.

Das Sensorsystem 8 errechnet also die Positions- und Orientierungswerte - definiert durch die drei Translationsfreiheitsgrade (X-, Y- und Z-Achse) und durch die drei Rotationsfreiheitsgrade - und ermittelt diese Daten an das Bildverarbeitungssystem, welche aus diesen Positions- und Orientierungsrohdaten durch Kalibrierung die echten Positions- und Orientierungsdaten errechnet, die dem jeweiligen Bildinhalt zugeordnet werden.

Die einzelnen Bilder werden von dem Ultraschallsystem 6 in einer vorgegebenen zeitlichen Folge (Sequenz) als analoge oder digitale Bild- oder Videosequenz an den Eingang des nachgeschalteten Bildverarbeitungssystems 1 geliefert. Mit dem Einschalten bzw. Aktivieren dieses Systems werden die Bilder, die beispielsweise Halbbilder oder Vollbilder sind, im Bildverarbeitungssystem 1 digitalisiert (bei analoger Videosequenz) oder aber in digitaler Form übernommen (bei digitaler Videosequenz) und mit dem zugehörigen Bild-Header bzw. den Header-Daten digital in einem Speicher des Bildverarbeitungssystems 1 abgelegt (Bildeinzug).

Der Bildeinzug am Bildverarbeitungssystem 1 wird durch den Benutzer der Vorrichtung ausgelöst (z.B. durch Betätigen eines Fußschalters). Ebenso wird der Bildeinzug beispielsweise durch den Benutzer oder aber automatisch durch das Bildverarbeitungssystem 1 dann beendet, wenn der von der Vielzahl der Einzelbilder mit jeweils zugehörigem Header gebildete dreidimensionale Datensatz eine vorgegebene Größe erreicht hat.

Ist beispielsweise diese vorgegebene Größe 30 Megabyte, so bedeutet dies, daß bei einer Größe jedes Einzelbildes von 256 x 256 Pixel und bei einer Grauwerttiefe von 8 Bit sowie bei einem Einzug von 50 Halbbildern pro Sekunde, das Speichervolumen nach etwa 8 Sekunden und nach einem Einzug von 457 Einzelbildern gefüllt ist und der Bildeinzug dann von dem Bildverarbeitungsgerät automatisch beendet wird. Die Verwendung einer Bildmaskierung ermöglicht den Einzug größerer Bildmengen durch eine Reduktion des Speicherpaltzbedarfs pro Einzelbild.

Da die Bildebenen der Einzelbilder durch die freie Führung des Ultraschallkopfes sehr unterschiedlich im Raum orientiert sind, erfolgt während des Bildeinzugs, d.h. in einer Datenerfassungs- oder akquisitionsphase zunächst eine Ablage der Einzelbilder als Rohdaten als Rohdatensatz 18 (Fig. 6). Nach Beendigung des Bildeinzugs erfolgt dann im Bildbearbeitungssystem 1 eine Transformation dieser Rohdaten unter Verwendung eines speziellen Programms auf ein gemeinsames Bezugssystem, beispielsweise kathesisches Koordinatensystem mit den Achsen X', Y' und Z' (Fig. 6), um so einen für die spätere Verwendung brauchbaren dreidimensionalen Datensatz 19 über das untersuchte Volumen zu erhalten.

Dieser dreidimensionale digitale Datensatz 19 ist grundsätzlich so aufgebaut, daß er eine Vielzahl von einzelnen Volumenelementen 20 (Voxel) enthält, die in diesem Datensatz 19 eine vorgegebene Lage in Bezug auf das gemeinsame Bezugssystem X'-, Y'- und Z'-Achse) aufweisen. Jeder Bildpunkt 20' der die Rohdaten 18 bildenden Bilder wird bei dieser Transformation in ein entsprechendes Voxel 20 dieses dreidimensionalen Datensatzes lagerichtig einsortiert. Ist bei der Bilderfassung ein bestimmter Bereich des zu untersuchenden Volumens nicht erfaßt worden, so kann bei der Transformation auch dem entsprechenden Voxel 20 kein Bildwert zugeordnet werden, d.h. der dreidimensionale Datensatz 19 würde dann Lücken enthalten, die aber durch Interpolation ausgefüllt werden können.

Es besteht aber auch die Möglichkeit, daß bei der Bilderfassung bestimmte Bereiche des zu überprüfenden Volumens zufällig mehrfach abgetastet wurden und daß dann bestimmte Bildwerte 20' überbestimmt sind. In diesem Fall erfolgt entweder für überbestimmte Bildwerte im Bildverarbeitungssystem 1 die Bildung eines Durchschnittswertes oder aber es wird nach einer Auswahlroutine nur ein Bildwert weiter verwendet.

Die erfindungsgemäße Vorrichtung eignet sich in besonders vorteilhafter Weise auch zur tomografischen Bilderfassung von bewegten Organen, insbesondere von Organen, deren Bewegung in direktem Bezug zum Herzschlag steht (z.B. Herzgefäße), und zwar wiederum mit freier Führung des Ultraschall-Wandlers.

Hier besteht grundsätzlich die Möglichkeit, einen statischen dreidimensionalen Datensatz von einem solchen Gewebe zu generieren und hierbei geometrische Verzerrungen, die sich aus der Bewegung, beispielsweise Herztätigkeit ergeben, zu vermeiden. In diesem Fall erfolgt der Einzug der Bilder durch das Bildverarbeitungssystem 1 synchron mit dem Herzzyklus bzw. mit der positiven oder negativen R-Spitze des Herzyklus der zu untersuchenden Person. Der Herzzyklus bzw. die R-Spitze werden mittels eines EKG-Gerätes 11 ermittelt, an welches über Sonden 12 an den Patient 15 angeschlossen ist. Durch die Triggerung erfolgt jeder Bildeinzug in einem genau definierten Zeitfenster innerhalb des Herzzyklus, d.h. jeder Bildeinzug erfolgt jeweils in einem definierten zeitlichen Abstand (Phasenlage) nach dem Auftreten der jeweiligen, zur Triggerung verwendeten R-Spitze im EKG-Zyklus. Hiermit sind dann alle im Bildverarbeitungssystem 1 abgelegten und die Rohdaten bildenden Einzelbilder phasensynchron mit dem Herzzyklus, so daß diese Einzelbilder zu einem dreidimensionalen, statischen Datensatz 19 umgewandelt werden können, der bzw. dessen einzelne Voxel 20 keine geometrischen Verzerrungen aufweisen.

Durch Änderung der Phasenlage des Zeitfensters für den Bildeinzug in bezug auf die triggernde R-Spitze können dreidimensionale tomografische Datensätze 19 von dem untersuchten Volumen erstellt werden, die dieses Volumen jeweils zu unterschiedlichen Zeiten im EKG-Zyklus wiedergeben.

Mit der erfindungsgemäßen Vorrichtung lassen sich weiterhin auch dynamische, dreidimensionale Datensätze 19 von dem untersuchten Volumen, nämlich von bewegten Organen usw. herstellen. In diesem Fall erfolgt der Einzug der Bilder am Bildverarbeitungssystem nicht synchron mit dem Herzzyklus, sondern unabhängig von diesem. Auch hier wird wiederum im Bildheader jedes Einzelbildes 21 der Meßwert, der die Phasenlage im EKG-Zyklus bestimmt, mit abgelegt.

Dieser dreidimensionale, dynamische Datensatz 19, der wiederum durch Transformation der Rohdaten 18 auf ein gemeinsames Bezugssystem erhalter, wird, besteht beispielsweise aus einer Vielzahl von Teil-Datensätzen 19', von denen jeder das gesamte zu untersuchende Volumen erfaßt und sich seinerseits aus einer Vielzahl von Volumenelementen 20 zusammensetzt, wobei die Teil-Datensätze 19' das untersuchte Volumen aber jeweils zu unterschiedlichen Zeitpunkten im EKG-Zyklus wiedergeben.

Die Erstellung des dynamischen, dreidimensionalen Datensatzes 19 erfolgt beispielsweise für einen kompletten EKG-Zyklus oder aber auch für nur einen Teil eines solchen Zyklus, wobei für die Generierung dieses Datensatzes 19 Bilder oder Bildwerte aus unterschiedlichen EKG-Zyklen verwendet werden, und zwar derart, daß nach oder bei der Transformation der Rohdaten 18 sämtliche vorhandenen Bildwerte aus unterschiedlichen EKG-Zyklen jeweils lagerichtig in die Voxel 20 des ihrer Phasenlage entsprechenden Teildatensatzes 19' eingeordnet werden.

Fehlen bei der Generierung der Teildatensätze 19' Bildwerte, weil bei der Bilderfassung Bereiche des untersuchten Volumens nicht erfaßt wurden, so können solche Lücken wiederum durch Interpolation von dem Bildverarbeitungssystem ausgefüllt. Sind mehrere Bildwerte pro Voxel 20 vorhanden, so wird diese Überbestimmung wiederum durch Bildung eines Durchschnittswertes oder durch Verwerfung von bestimmten Bildwerten oder durch Kombination beider Verfahren behoben.

Dreidimensionale Datensätze 19 können als statische oder dynamische Datensätze auch von Organen oder Körperteilen erstellt werden, die einer passiven Bewegung, beispielsweise durch Atmung unterworfen sind. Hierfür ist an das Bildverarbeitungssystem 1 das Gerät 13 angekoppelt, welches die Atemlage bzw. den Respirationszyklus des Patienten erfaßt. Dieser Respirationszyklus, der in der Fig. 2 im Diagramm b wiedergegeben ist, besteht jeweils aus der Expirations-Phase (Ausatmungsphase) und der Inspirations-Phase (Einatmungsphase). Mit diesem Respirationszyklus, beispielsweise mit den dortigen Spitzen A kann zur Erstellung eines durch die passive Bewegung unverzerrten statischen dreidimensionalen Datensatzes 19 das Bildverarbeitungssystem 1 wieder getriggert bzw. synchronisiert werden, und zwar derart, daß der Bildeinzug jeweils nur während eines vorbestimmten Fensters im Atemzyklus erfolgt. Grundsätzlich kann die Steuerung auch so erfolgen, daß der Bildeinzug nur dann erfolgt, wenn die Atemlage nicht aktiviert ist, also kein Respirationssignal vorliegt.

Grundsätzlich kann unter Berücksichtigung der Atemlage auch ein dynamischer, dreidimensionaler Datensatz generiert werden, wobei dann das Respirationszyklus in der Weise verwendet wird, wie dies vorstehend für den EKG-Zyklus bei der Erstellung des dynamischen Datensatzes 19 beschrieben wurde.

Die Fig. 3 - 5 zeigen in einem Ablaufdiagramm mehr im Detail die Arbeitsweise der erfindungsgemäßen Vorrichtung.

Die Fig. 3 betrifft die anfängliche Einstellung insbesondere des Bildverarbeitungssystems 1 vor der Erfassung der Bilder (Datenakquisition). Zunächst wird entschieden, ob das Bezugssystem, d.h. die räumliche Zuordnung zwischen der Bildebene des Ultraschallkopfes 7 und dem Empfänger 10 neu kalibriert werden muß, beispielsweise wegen Verwendung eines neuen Ultraschallkopfes 7, der im System noch nicht benutzt wurde.

Ist ein neues Bezugssystem notwendig, wird eine entsprechende Kalibrierung vorgenommen. Ist die Verwendung eines bekannten Bezugssystemes möglich, kann auf diese bzw. diese Kalibrierung zurückgegriffen werden. Im Anschluß daran erfolgt eine Definition von möglichen Auswahlkriterien. Soll eine dynamische Datenerfassung unter Berücksichtigung der Herztätigkeit erfolgen, so wird ein Schwellwert bezüglich der Größe des RR-Intervalls eingestellt. Diese Einstellung ist nicht notwendig, wenn eine dynamische Datenerfassung nicht gewünscht ist. Es erfolgt dann die Entscheidung, ob die Atemlage berücksichtigt werden soll. Ist dies der Fall, erfolgt eine Definition für den Schwellwert bezüglich der Atemlage, beispielsweise durch Einstellung eines maximalen und minimalen Schwellwertes für die Dauer eines Respiratonszyklus. Eine Definition des Schwellwertes entfällt, wenn die Atemlage nicht berücksichtigt werden soll.

Nach dieser Voreinstellung erfolgt entsprechend Fig. 4 die Datenerfassung bzw. Akquisition. Zunächst werden die von dem Ultraschallkopf 7 gelieferten Ultraschallbilder digitalisiert. Dann wird bei einer dynamischen Datenerfassung die Phasenlage aus dem EKG-Zyklus und/oder die Phasenlage auf dem Respirationszyklus bestimmt, wobei diese Bestimmung entfällt, wenn eine dynamische Datenakquisition bzw. eine Datenakquisition unter Berücksichtigung der Atemlage nicht gewünscht sind.

In jedem Fall erfolgt bei jedem Bildeinzug die Positionserkennung. Die erfaßten Bilddaten werden dann zusammen mit den weiteren Daten als Rohdatensatz 18 abgelegt, wobei die weiteren Daten (Meßwert für die Phasenlage im EKG-Zyklus, Meßwert für die Phasenlage im Respirationszyklus und Positionsdaten) im Bildheader abgelegt werden.

Anschließend erfolgt eine Überprüfung der Daten anhand der im Verfahrensschritt der Fig. 3 getroffenen Auswahlkriterien. Solche Daten, die diesen Auswahlkriterien nicht entsprechen, werden verworfen und entfernt.

Die Datenakquisition ist beendet, wenn das zu überprüfende tomografische Volumen komplett ist, was entweder automatisch durch das Bildverarbeitungssystem nach einem Zeitkriterium festgestellt oder aber vom Benutzer bestätigt wird.

Sofern das tomografische Volumen nicht komplett ist, werden die Verfahrensschritte der Fig. 4 erneut durchgeführt.

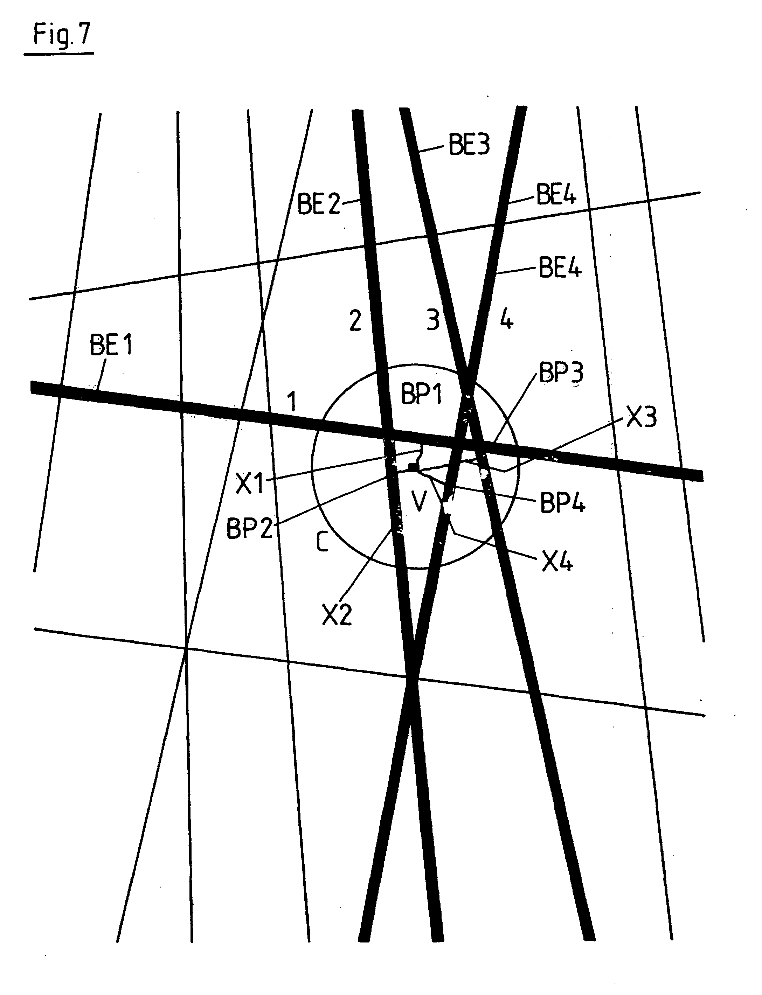

Die Fig. 5 - 7 erläutern die Transformation der Rohdaten 18 in den Datensatz 19. Wie oben bereits ausgeführt wurde, werden mit Hilfe des Sensors 17 zunächst Einzelbilder, d.h. zweidimensionale Einzelbilder 21 in unterschiedlichen Bildebenen (in Abhängigkeit von der jeweiligen Positionierung des Sensors 17 erzeugt. Die Einzelbilder 21 setzen sich jeweils aus einer Vielzahl von Bildpunkten 20' mit jeweils unterschiedlichen Bild- oder Grauwerten zusammen. Bei der Bildung des dreidimensionalen Datensatzes 19 aus den Einzelbildern 21 erfolgt das Überführen bzw. Transformieren (lagerichtiges Einsortieren) der einzelnen Bilddaten 20' bzw. Grauwerte in das neue, gemeinsame Bezugssystem, welches beispielsweise ein kartesisches Koordinatensystem ist. Durch Interpolation erfolgt hierbei auch das Ausfüllen von Lücken. Diese Transformieren wird nachfolgend anhand der Fig. 7 noch näher erläutert. Diese Verfahrensschritte werden unter Verwendung der Kalibrierdaten durchgeführt.

Sofern eine dynamische Datenerfassung bzw. -Akquisition vorgenommen wird, erfolgt eine Interpolation von zeitlichen Lücken, insbesondere auch unter Verwendung von Bilddaten aus unterschiedlichen EKG-Zyklen. Erfolgte keine dynamische Akquisition, so entfällt dieser Verfahrensschritt.

Anschließend erfolgt die Entscheidung, ob die Atemlage berücksichtigt werden soll oder nicht. Ist letzteres der Fall, so werden beispielsweise solche Daten eliminiert und verworfen, bei denen die Atemtätigkeit einen vorgegebenen Schwellwert übersteigt.

Die gewonnenen Daten werden schließlich so abgelegt, daß sie für eine zweidimensionale und/oder dreidimensionale Darstellung sowie auch für eine zweidimensionale und/oder dreidimensionale Quantifizierung verwendet werden können.

Die Figur 7 zeigt in vereinfachter schematischer Darstellung verschiedene im zu untersuchenden Volumen erzeugte zweidimensionale Einzelbilder 21 bzw. deren Bildebenen BE1 - BE4. Der einfacheren Darstellung wegen ist davon ausgegangen, daß diese Bildebenen jeweils senkrecht zur Zeichenebene der Figur 7 liegen. Dargestellt ist weiterhin ein Voxel 20, welches ebenso wie alle übrigen Voxel hergestellt wird, die den transformierten dreidimensionalen Datensatz 19 bilden. Bei der Transformation der Rohdaten 18 in den Datensatz 19, d.h. bei der Bildung der Voxel 20 im Bildverarbeitungssystem 1 zunächst beispielsweise nach einer vorgegebenen Programmroutine ein Voxel 20 bzw. dessen Lage (Koordinaten) im Datensatz 19 ausgewählt und dann anschließend für dieses Voxel ein Bild- oder Grauwert nach einer vorgegebenen Funktion oder nach einem vorgegebenen Algorithmus gebildet, und zwar aus den Grauwerten derjenigen Bildpunkte 20', die in den Bildebenen BE1 - BE4 dem Voxel 20 jeweils am nächsten liegen und deren Abstand vom jeweiligen Voxel 20 einen vorgegebenen, in der Figur 7 mit C bezeichneten Abstand nicht übersteigt. Der Wert C ist entweder durch das System selbst fest vorgegeben oder aber kann durch den Verwender des Systems zumindest in gewissen Grenzen eingestellt werden. Bevorzugt erfolgt die Bildung des Bild- oder Grauwertes für das jeweils zu bildende Voxel 20 durch Bildung eines gewichteten Summenwertes (weighted sum oder eines gewichteten Mittelwertes.

Berücksichtigt werden dabei, wie ausgeführt, jeweils diejenigen Bildpunkte jeder Bildebene BE1-BE4, die den jeweiligen Voxel 20 am nächsten liegen und deren Abstand von dem Voxel 20 den maximalen Abstand C nicht übersteigt. Diese Bildpunkte sind in der Figur 7 mit BP1 (auf der Bildebene BE1), BP2 (auf der Bildebene BE2), BP3 (auf der Bildebene BE3) und BP4 (auf der Bildebene BE4) bezeichnet. Die Abstände zwischen dem Voxel 20 und dem jeweiligen Bildpunkt BP1 - BP4 sind jeweils mit X1, X2, X3 und X4 bezeichnet. Unter Berücksichtigung dieses maximal zulässigen Abstandes C und der Abstände X1-X4 ergeben sich für die Bestimmung der Bild- und Grauwerte der Bildpunkte BP1 - BP4 folgende Gewichtungen:

$$W1 = C - X1$$

$$W2 = C - X2$$

$$W3 = C - X3$$

$$W4 = C - X4.$$

Die Bild- oder Grauwerte der Bildpunkte BP1 - BP4 sind jeweils G1 für den Bildpunkt BP1, G2 für den Bildpunkt BP2 usw.

Für den Grauwert GV des jeweiligen Voxels gilt dann:

$$GV = (G1W1 + G2W2 + G3W3 + G4W4)/(W1 + W2 + W3 + W4).$$

Es versteht sich, daß die Bildung des Wertes GV für sämtliche Voxel 20 erfolgt, und zwar in der Weise, daß ein vollständiger, dreimdimensionaler Datensatz 19 erzeugt wird.

**Patentansprüche**

1. Verfahren zur Generierung eines diagnostisch verwertbaren dreidimensionalen Bild-Datensatzes (19) unter Verwendung eines Ultraschallgerätes (6) mit frei geführtem Ultraschall-Kopf (7) zur Erzeugung einer Sequenz einer Vielzahl von Ultraschall-Einzelbildern in unterschiedlichen Bildebenen (BE1, BE2, BE3, BE4) des zu untersuchenden Volumens, unter Verwendung eines an das Ultraschallgerät (6) angeschlossenen Bildverarbeitungssystems (1), dem die Sequenz der generierten Ultraschallbilder zugeführt werden, unter Verwendung eines Positions-Sensorsystems (8), welches die Position und Orientierung des Ultraschallkopfes (7) und damit die räumliche Lage der Bildebene des jeweils generierten Ultraschallbildes bestimmt, und zwar in bezug auf die drei Translations- und Rotationsfreiheitgrade, wobei diese Positions- und Orientierungsdaten des Sensorsystems ebenfalls an das Bildverarbeitungssystem (1) übertragen werden, welches aus Bilddaten der Ultraschall-Einzelbilder und den Positions- und Orientierungsdaten den dreidimensionalen, das untersuchte Volumen tomografisch erfassenden Datensatz (19) generiert, welcher aus einer Vielzahl von Voxeln (20) mit jeweils einem zugeordneten Bildwert, beispielsweise einem zugeordneten Grauwert, besteht und in welchem die Lage der Voxel (20) auf ein gemeinsames Bezugssystem, vorzugsweise auf ein kartesisches Koordinatensystem bezogen sind, wobei zur dreidimensionalen tomografischen Bilderfassung eines zu untersuchenden Volumens als Sensorsystem (8) ein elektromagnetisches System verwendet ist, dessen Empfänger (10) am Ultraschallkopf (7) vorgesehen ist, wobei die Ultraschall-Einzelbilder in einer eine Datenakquisition bildenden Phase zunächst im Bildverarbeitungssystem (1) als Rohdaten jeweils zusammen mit einem Bildheader abgelegt werden, der zumindest die Positions- und Orientierungsdaten enthält, wobei das Bildverarbeitungssystem (1) den Rohdatensatz (18) in den dreidimensionalen Datensatz (19) transformiert, **dadurch gekennzeichnet,**
**daß** bei der Transformation der Rohdaten (18) in den Datensatz (19) für jedes Voxel (20) der zugehörige Bildwert durch Bildung eines gewichteten Summenwertes oder eines gewichteten Mittelwertes erzeugt wird,
und zwar nach der Formel

$$GV = \{G1*(C - X1) + G2*(C - X2) + ..... + Gn*C - Xn)\}/ \{(C - X1) + (C - X2)$$

$$+ ..... + (C - Xn)\},$$

wobei :

GV der Bildwert des jeweiligen Voxels (20) ist,
G1 ..... Gn die Bildwerte der dem jeweiligen Voxel (20) am nächsten liegenden Bildpunkte beachbarter Einzelbilder sind, und
X1, X2,...... Xn der jeweilige Abstand dieser Bildpunkte der Einzelbilder von dem jeweiligen Voxel (20) ist, wobei der jeweilige Abstand einen maximalen Abstand C nicht übersteigt.

**2.** Verfahren nach Anspruch 1, **dadurch gekennzeichnet, daß** bei der Transformation zunächst nach einer vorgegebenen Programmroutine das jeweilige Voxel (20) bzw. dessen Lage ausgewählt und dann anschließend der Bildwert des Voxels (20) gebildet wird.

**3.** Verfahren nach einem der vorhergehenden Ansprüche, **dadurch gekennzeichnet, daß** der Empfänger oder die Empfängerspulen dieses Sensorsystems am Ultraschallkopf (7) oder an dem am Ultraschallkopf befestigten Halter (17) vorgesehen sind, und daß das Bilderfassungssystem (1) die von dem Sensorsystem gelieferten Positions- und Orientierungsdaten unter Verwendung von Parametern kalibriert, die den räumlichen Bezug zwischen dem Empfänger (11) des Sensorsystems und dessen Orientierung im Raum und der Lage der Bildebene des Ultraschallkopfes (7) berücksichtigt,
wobei beispielsweise die Kalibrierung der Positions- und Orientierungsdaten in einer auf die Phase der Datenakquisition folgenden Nachverarbeitungsphase erfolgt, und/oder wobei beispielsweise die Kalibrierung der Positions- und Orientierungsdaten während der Phase der Datenakquisition erfolgt und vorzugsweise die kalibrierten Positions- und Orientierungsdaten im jeweiligen Bild-Header abgelegt werden.

**4.** Verfahren nach einem der vorhergehenden Ansprüche, **gekennzeichnet durch** ein an das Bildverarbeitungssystem (1) angeschlossenes EKG-Gerät zur Erzeugung eines einen EKG-Zyklus definierenden Signals, insbesondere eines die R-Spitzen des EKG-Zyklus repräsentierenden Signals, wobei das Bildverarbeitungssystem (1) Mittel aufweist, um im Bild-Header einen Wert, der die Phasenlage zwischen einem definierten Zeitpunkt des EKG-Zyklus, beispielsweise der R-Spitze, und dem Einzug eines Ultraschall-Einzelbildes am Bildverarbeitungssystem repräsentiert.

**5.** Verfahren nach einem der vorhergehenden Ansprüche, **dadurch gekennzeichnet, daß** zum Generieren eines dreidimensionalen, statischen Datensatzes das Einziehen der Ultraschall-Bilder am Bildverarbeitungssystem (1) synchron mit dem EKG-Zyklus jeweils in einem vorgegebenen Zeitfenster dieses Zyklus erfolgt, und/oder
daß zum Generieren eines dreidimensionalen dynamischen Datensatzes (19, 19') das Einziehen der Ultraschall-Einzelbilder am Bildverarbeitungssystem (1) ständig erfolgt, und daß das Bildverarbeitungssystem Mittel aufweist, um die Bildwerte jeweils der Phasenlage im Herzzyklus entsprechend im dreidimensionalen Datensatz (19, 19') abzulegen, und/oder
daß ein Gerät (13) zur Erfassung des Respirations-Zyklus verwendet ist, und daß im Bildheader ein Meßwert, der die Phasenlage im Respirationszyklus bestimmt, abgelegt wird.

**6.** Vorrichtung zur Generierung eines diagnostisch verwertbaren dreidimensionalen Bild-Datensatzes (19) unter Verwendung eines Ultraschallgerätes (6) mit frei geführtem Ultraschall-Kopf (7) zur Erzeugung einer Sequenz einer Vielzahl von Ultraschall-Einzelbildern in unterschiedlichen Bildebenen (BE1, BE2, BE3, BE4) des zu untersuchenden Volumens, unter Verwendung eines an das Ultraschallgerät (6) angeschlossenen Bildverarbeitungssystems (1), dem die Sequenz der generierten Ultraschallbilder zugeführt werden, unter Verwendung eines Positions-Sensorsystems (8), welches die Position und Orientierung des Ultraschallkopfes (7) und damit die räumliche Lage der Bildebene des jeweils generierten Ultraschallbildes bestimmt, und zwar in bezug auf die drei Translations- und Rotationsfreiheitgrade, wobei diese Positions- und Orientierungsdaten des Sensorsystems ebenfalls an das Bildverarbeitungssystem (1) übertragen werden, welches aus Bilddaten der Ultraschall-Einzelbilder und den Positions- und Orientierungsdaten den dreidimensionalen, das untersuchte Volumen tomografisch erfassenden Datensatz (19) generiert, welcher aus einer Vielzahl von Voxeln (20) mit jeweils einem zugeordneten Bildwert, beispielsweise einem zugeordneten Grauwert, besteht und in welchem die Lage der Voxel (20) auf ein gemeinsames Bezugssystem, vorzugsweise auf ein kartesisches Koordinatensystem bezogen sind, wobei zur dreidimensionalen tomografischen Bilderfassung eines zu untersuchenden Volumens als Sensorsystem (8) ein elektromagnetisches System verwendet ist, dessen Empfänger (10) am Ultraschallkopf (7) vorgesehen ist, wobei die Ultraschall-Einzelbilder in einer eine Datenakquisition bildenden Phase zunächst im Bildverarbeitungssystem (1) als Rohdaten jeweils zusammen mit einem Bildheader abgelegt werden, der zumindest die Positions- und Orientierungsdaten enthält, und wobei das Bildverarbeitungssystem (1) den Rohdatensatz (18) in den dreidimensionalen Datensatz (19) transformiert, **gekennzeichnet durch** Mittel, die bei der Transformation der Rohdaten (18) in den Datensatz (19) für jedes Voxel (20) den zugehörigen Bildwert **durch** Bildung eines gewichteten Summenwertes oder eines gewichteten Mittelwertes erzeugen,
und zwar nach der Formel

$$GV = \{G1*(C - X1) + G2*(C - X2) + ........ + Gn*C - Xn)\}/ \{(C - X1) +(C - X2)$$

$$....... + (C - Xn)\},$$

wobei:

GV der Bildwert des jeweiligen Voxels (20) ist,
G1 ..... Gn die Bildwerte der dem jeweiligen Voxel (20) am nächsten liegenden Bildpunkte beachbarter Einzelbilder sind, und
X1, X2 ...... Xn der jeweilige Abstand dieser Bildpunkte der Einzelbilder von dem jeweiligen Voxel (20) ist, wobei der jeweilige Abstand einen maximalen Abstand C nicht übersteigt.

7. Vorrichtung nach Anspruch 6, **dadurch gekennzeichnet, daß** bei der Transformation zunächst nach einer vorgegebenen Programmroutine das jeweilige Voxel (20) bzw. dessen Lage ausgewählt und dann anschließend der Bildwert des Voxels (20) gebildet wird.

8. Vorrichtung nach einem der Ansprüche 6 oder 7, **dadurch gekennzeichnet, daß** der Empfänger oder die Empfängerspulen dieses Sensorsystems am Ultraschallkopf (7) oder an dem diesen Ultraschallkopf tragenden Halter (17) vorgesehen sind, und daß das Bilderfassungssystem (1) Mittel aufweist, um die von dem Sensorsystem gelieferten Positions- und Orientierungsdaten unter Verwendung von Parametern zu kalibrieren, die den räumlichen Bezug zwischen dem Empfänger (11) des Sensorsystems und dessen Orientierung im Raum und der Lage der Bildebene des Ultraschallkopfes (7) berücksichtigt,
wobei beispielsweise die Kalibrierung der Positions- und Orientierungsdaten in einer auf die Phase der Datenakquisition folgenden Nachverarbeitungsphase erfolgt, oder
wobei beispielsweise die Kalibrierung der Positions- und Orientierungsdaten während der Phase der Datenakquisition erfolgt, und daß vorzugsweise die kalibrierten Positions- und Orientierungsdaten im jeweiligen Bild-Header abgelegt werden.

9. Vorrichtung nach einem der Ansprüche 6, 7 oder 8, **gekennzeichnet durch** ein an das Bildverarbeitungssystem (1) angeschlossenes EKG-Gerät zur Erzeugung eines einen EKG-Zyklus definierenden Signals, insbesondere eines die R-Spitzen des EKG-Zyklus repräsentierenden Signals, wobei das Bildverarbeitungssystem (1) Mittel aufweist, um im Bild-Header einen Wert, der die Phasenlage zwischen einem definierten Zeitpunkt des EKG-Zyklus, beispielsweise der R-Spitze, und dem Einzug eines Ultraschall-Einzelbildes am Bildverarbeitungssystem repräsentiert.

10. Vorrichtung nach einem der Ansprüche 6 bis 9, **dadurch gekennzeichnet, daß** zum Generieren eines dreidimensionalen, statischen Datensatzes das Einziehen der Ultraschall-Bilder am Bildverarbeitungssystem (1) synchron mit dem EKG-Zyklus jeweils in einem vorgegebenen Zeitfenster dieses Zyklus erfolgt, und/oder
daß zum Generieren eines dreidimensionalen dynamischen Datensatzes (19, 19') das Einziehen der Ultraschall-Einzelbilder am Bildverarbeitungssystem (1) ständig erfolgt, und daß das Bildverarbeitungssystem Mittel aufweist, um die Bildwerte jeweils der Phasenlage im Herzzyklus entsprechend im dreidimensionalen Datensatz (19, 19') abzulegen, und/oder
daß ein Gerät (13) zur Erfassung des Respirations-Zyklus vorgesehen ist, und daß im Bildheader ein Meßwert, der die Phasenlage im Respirationszyklus bestimmt, abgelegt wird.

## Claims

1. Method for generating a three-dimensional image data record (19) that can be used for diagnostics (19), using an ultrasound unit (6) with freely held ultrasound head (7) to create a sequence of numerous ultrasonic individual images in different image planes (BE1, BE2, BE3, BE4) of the volume to be investigated, using an image processing system (1) which is connected to the ultrasound unit (6), and to which the sequence of generated ultrasonic images is supplied, using a position sensor system (8) which establishes the position and orientation of the ultrasound head (7) and thus the spatial location of the image plane of the respectively generated ultrasonic image, and in fact in relation to the three translational and rotational degrees of freedom, wherein these position and orientation data of the sensor system are likewise transmitted to the image processing system (1) which, from image data of the ultrasonic individual images and the position and orientation data, generates the three-dimensional data record (19) which tomographically records the volume that is examined, and which comprises numerous voxels (20) with respectively one assigned image value, for example an assigned grey-scale value, and in which the position of the voxels (20) is related to a common reference system, preferably to a Cartesian co-ordinate system, wherein for three-dimensional tomographic image recording of a volume that is to be examined, an electromagnetic system is used as the sensor system (8), whose receiver (10) is provided on the ultrasound head (7), wherein in a phase that forms the data acquisition, the ultrasonic individual images are initially filed in the image processing system (1) as raw data respectively together with an image header which contains at least the position and orientation data, wherein the, image processing system (1) transforms the raw data record (18) into the three-dimensional data record (19),
**characterised in that**
in the transformation of the raw data (18) into the data record (19), for each voxel (20) the corresponding image value is produced through the formation of a weighted cumulative value or of a weighted mean value, and in fact according to the formula

$$GV = \{ G1 * (C-X1) + G2 * (C-X2) + ...... + Gn * (C-Xn) \} / \{ (C-X1) + (C-X2) + ..... + (C-Xn) \},$$

where
GV is the image value of the respective voxel (20),
G1 ..... Gn are the image values of those pixels of adjacent individual images which lie closest to the respective voxel (20), and
X1, X2, ..... Xn is the respective distance of these pixels of the individual images from the respective voxel (20), wherein the respective distance does not exceed a maximum distance C.

2. Method in accordance with claim 1, **characterised in that** in the transformation, first of all the respective voxel (20) or its position is selected in accordance with a given program routine, and then subsequently the image value of the voxel (20) is formed.

3. Method in accordance with one of the preceding claims, **characterised in that** the receiver or the receiver coils of this sensor system are provided on the ultrasound head (7) or on the holder (17) fastened to the ultrasound head, and that the image recording system (1) calibrates the position and orientation data supplied by the sensor system, using parameters which take account of the spatial relation between the receiver (11) of the sensor system and its orientation in space and the position of the image plane of the ultrasound head (7),
wherein for example the calibration of the position and orientation data takes place in an after-processing phase that follows the phase of data acquisition,
and/or wherein for example the calibration of the position and orientation data takes place during the phase of data acquisition and preferably the calibrated position and orientation data are filed in the respective image header.

4. Method in accordance with one of the preceding claims, **characterised by** an ECG device that is connected to the image processing system (1), to produce a signal that defines an ECG cycle, in particular a signal that represents the R-peaks of the ECG cycle, wherein the image processing system (1) has means for [*storing*] in the image header a value which represents the phase position between a defined point in time of the ECG cycle, for example of the R-peak, and the calling in of an ultrasonic individual image on the image processing system.

5. Method in accordance with one of the preceding claims, **characterised in that** to generate a three-dimensional static data record, the calling in of the ultrasonic images on the image processing system (1) takes place synchronously with the ECG cycle, respectively in a given time window of this cycle, and/or
that to generate a three-dimensional data record (19, 19'), the calling in of the ultrasonic individual images on the image processing system (1) takes place continually, and that the image processing system has means for filing the image values respectively of the phase position in the cardiac cycle accordingly in the three-dimensional data record (19, 19'), and/or
that a device (13) is used for recording the respiration cycle, and that in the image header a measured value is filed which determines the phase position in the respiration cycle.

6. Device for generating a three-dimensional image data record (19) that can be used for diagnostics (19), using an ultrasound unit (6) with freely held ultrasound head (7) to create a sequence of numerous ultrasonic individual images in different image planes (BE1, BE2, BE3, BE4) of the volume to be investigated, using an image processing system (1) which is connected to the ultrasound unit (6), and to which the sequence of generated ultrasonic images is supplied, using a position sensor system (8) which establishes the position and orientation of the ultrasound head (7) and thus the spatial location of the image plane of the respectively generated ultrasonic image, and in fact in relation to the three translational and rotational degrees of freedom, wherein these position and orientation data of the sensor system are likewise transmitted to the image processing system (1) which, from image data of the ultrasonic individual images and the position and orientation data, generates the three-dimensional data record (19) which tomographically records the volume that is examined, and which comprises numerous voxels (20) with respectively one assigned image value, for example an assigned grey-scale value, and in which the position of the voxels (20) is related to a common reference system, preferably to a Cartesian co-ordinate system, wherein for three-dimensional tomographic image recording of a volume that is to be examined, an electromagnetic system is used as the sensor system (8), whose receiver (10) is provided on the ultrasound head (7), wherein in a phase that forms the data acquisition, the ultrasonic individual images are initially filed in the image processing system (1) as raw data respectively together with an image header which contains at least the position and orientation data, wherein the image processing system (1) transforms the raw data record (18) into the three-dimensional data record (19),
**characterised by** means which, in the transformation of the raw data (18) into the data record (19), produce for each voxel (20) the corresponding image value through the formation of a weighted cumulative value or of a weighted mean value, and in fact according to the formula

$$GV = \{ G1 * (C-X1) + G2 * (C-X2) + ..... + Gn * (C-Xn) \} / \{ (C-X1) + (C-X2) + ..... + (C-Xn) \},$$

where
GV is the image value of the respective voxel (20),
G1 ..... Gn are the image values of the pixels of adjacent individual images which lie closest to the respective voxel (20), and
X1, X2, ..... Xn is the respective distance of these pixels of the individual images from the respective voxel (20), wherein the respective distance does not exceed a maximum distance C.

7. Device in accordance with claim 6, **characterised in that** in the transformation, first of all the respective voxel (20) or its position is selected in accordance with a given program routine, and then subsequently the image value of the voxel (20) is formed.

8. Device in accordance with one of the claims 6 or 7, **characterised in that** the receiver or the receiver coils of this sensor system are provided on the ultrasound head (7) or on the holder (17) that bears this ultrasound head, and that the image recording system (1) has means for calibrating the position and orientation data supplied by the sensor system, using parameters which take account of the spatial relation between the receiver (11) of the sensor system and its orientation in space and the position of the image plane of the ultrasound head (7),
wherein for example the calibration of the position and orientation data takes place in an after-processing phase that follows the phase of data acquisition, or
wherein for example the calibration of the position and orientation data takes place during the phase of data acquisition, and that preferably the calibrated position and orientation data are filed in the respective image header.

**9.** Device in accordance with one of the claims 6, 7 or 8, **characterised by** an ECG machine that is connected to the image processing system (1), to produce a signal that defines an ECG cycle, in particular a signal that represents the R-peaks of the ECG cycle, wherein the image processing system (1) has means for [*storing*] in the image header a value which represents the phase position between a defined point in time of the ECG cycle, for example of the R-peak, and the calling in of an ultrasonic individual image on the image processing system.

**10.** Device in accordance with one of the claims 6 to 9, **characterised in that** to generate a three-dimensional static data record, the calling in of the ultrasonic images on the image processing system (1) takes place synchronously with the ECG cycle, respectively in a given time window of this cycle, and/or
that to generate a three-dimensional data record (19, 19'), the calling in of the ultrasonic individual images on the image processing system (1) takes place continually, and that the image processing system has means for filing the image values respectively of the phase position in the cardiac cycle accordingly in the three-dimensional data record (19, 19'), and/or
that a device (13) is provided for recording the respiration cycle, and that in the image header a measured value is filed which determines the phase position in the respiration cycle.

## Revendications

**1.** Procédé de génération d'un jeu (19) de données d'images tridimensionnelles utilisables en diagnostic en mettant en oeuvre un appareil à ultrasons (6) comprenant une tête à ultrasons (7) à guidage libre pour la production d'une séquence d'une pluralité d'images individuelles ultrasoniques dans différents plans images (BE1, BE2, BE3, BE4) du volume à examiner, en utilisant un système de traitement d'images (1) raccordé à l'appareil à ultrasons (6), système auquel la séquence des images ultrasoniques générées est acheminée, en utilisant un système capteur de position (8), qui détermine la position et l'orientation de la tête à ultrasons (7) et, par suite, la position spatiale du plan image de l'image ultrasonique respectivement générée, et ce, par rapport aux trois degrés de liberté en translation et en rotation, où ces données de position et d'orientation du système capteur sont également transmises au système de traitement d'images (1), qui génère, à partir de données des images individuelles ultrasoniques et de données de position et d'orientation, le jeu de données tridimensionnel (19) saisissant par voie tomographique le volume examiné, qui est constitué d'une pluralité de voxels (20) avec, respectivement, une image correspondante, par exemple une valeur de gris correspondante, et dans lequel les positions des voxels (20) sont rapportées à un système de référence commun, de préférence à un système cartésien de coordonnées, où l'on utilise, pour la saisie tomographique d'images en trois dimensions d'un volume à examiner, comme système capteur (8) un système électromagnétique dont le récepteur (10) est ménagé sur la tête à ultrasons (7), où les images individuelles ultrasoniques sont déposées, dans une phase formant une acquisition de données, tout d'abord dans le système de traitement d'images (1) comme données brutes, respectivement, conjointement avec une en-tête d'image qui contient au moins les données de position et d'orientation, le système de traitement d'images (1) transformant le jeu d'images brutes (18) en jeux de données tridimensionnelles (19),
**caractérisé en ce que**
lors de la transformation des données brutes (18) en jeu de données (19), pour chaque voxel (20), la valeur d'image correspondante est produite par formation d'une valeur de sommation pondérée ou d'une valeur moyenne pondérée, selon la formule :

$$GV = \{G1*(C - X1) + G2*(C - X2) + ..... + Gn*(C-Xn)\} / \{(C - X1) + (C - X2) + ..... + (C - Xn)\}$$

où
GV est la valeur d'image du voxel respectif (20),

G1 ....... Gn sont les valeurs d'images des points d'images individuelles observables se trouvant le plus près du voxel respectif (20), et

X1, X2, ....... Xn la distance respective de ces points des images individuelles par rapport au voxel respectif (20), la distance respective ne dépassant pas une distance maximale C.

**2.** Procédé selon la revendication 1, **caractérisé en ce que**, lors de la transformation, on choisit tout d'abord, selon un programme prédéterminé, le voxel respectif (20) ou sa position et, ensuite, on forme la valeur d'image du voxel (20).

**3.** Procédé selon l'une quelconque des revendications précédentes, **caractérisé en ce que** le récepteur ou les bobines réceptrices de ce système capteur sont aménagés sur la tête à ultrasons (7) ou sur le support (17) fixé à la tête à ultrasons, et **en ce que** le système de saisie d'images (1) calibre les données de position et d'orientation délivrées par le système capteur en utilisant des paramètres qui font intervenir la référence spatiale entre le récepteur (11) du système capteur et son orientation dans l'espace et la position du plan image de la tête à ultrasons (7),

où, par exemple, le calibrage des données de position et d'orientation se fait dans une phase de post-traitement suivant la phase d'acquisition de données et/ou, par exemple, le calibrage des données de position et d'orientation se fait au cours de la phase d'acquisition de données et, de préférence, les données calibrées de position et d'orientation sont stockées dans l'en-tête d'image respective.

**4.** Procédé selon l'une quelconque des revendications précédentes, **caractérisé par** un appareil ECG raccordé au système de traitement d'images (1) pour produire un signal définissant un cycle ECG, en particulier un signal représentant les pointes R du cycle ECG, où le système de traitement d'images (1) présente des moyens pour stocker dans l'en-tête d'image une valeur qui représente la position de phase entre un moment défini du cycle ECG, par exemple les pointes R, et l'introduction d'une image individuelle ultrasonique sur le système de traitement d'images.

**5.** Procédé selon l'une quelconque des revendications précédentes, **caractérisé en ce que**, pour générer un jeu de données statiques tridimensionnelles, l'introduction des images ultrasoniques sur le système de traitement d'images (1) se fait en synchronisme avec le cycle ECG, respectivement, dans une fenêtre temporelle prédéterminée de ce cycle, et/ou **en ce que**, pour générer un jeu (19, 19') de données dynamiques tridimensionnelles, l'introduction des images individuelles ultrasoniques (1) se fait en continu sur le système de traitement d'images (1) et/ou **en ce que** le système de traitement d'images présente des moyens pour stocker les valeurs d'images dans le jeu de données tridimensionnelles (19, 19'), respectivement, en fonction de la position de phase dans le cycle cardiaque, et/ou **en ce qu'**on utilise un appareil (13) pour saisir le cycle de respiration et **en ce que** l'on stocke dans l'en-tête d'image une valeur de mesure qui détermine la position de phase dans le cycle respiratoire.

**6.** Dispositif de génération d'un jeu (19) de données d'images tridimensionnelles utilisables en diagnostic en mettant en oeuvre un appareil à ultrasons (6) comprenant une tête à ultrasons (7) à guidage libre pour la production d'une séquence d'une pluralité d'images individuelles ultrasoniques dans différents plans images (BE1, BE2, BE3, BE4) du volume à examiner, en utilisant un système de traitement d'images (1) raccordé à l'appareil à ultrasons (6), système auquel la séquence des images ultrasoniques générées est acheminée, en utilisant un système capteur de position (8), qui détermine la position et l'orientation de la tête à ultrasons (7) et, par suite, la position spatiale du plan image de l'image ultrasonique respectivement générée, et ce, par rapport aux trois degrés de liberté en translation et en rotation, où ces données de position et d'orientation du système capteur sont également transmises au système de traitement d'images (1), qui génère, à partir de données des images individuelles ultrasoniques et de données de position et d'orientation, le jeu de données tridimensionnel (19) saisissant par voie tomographique le volume examiné, qui est constitué d'une pluralité de voxels (20) avec, respectivement, une image correspondante, par exemple une valeur de gris correspondante, et dans lequel les positions des voxels (20) sont rapportées à un système de référence commun, de préférence à un système cartésien de coordonnées, où l'on utilise, pour la saisie tomographique d'images en trois dimensions d'un volume à examiner, comme système capteur (8) un système électromagnétique dont le récepteur (10) est ménagé sur la tête à ultrasons (7), où les images individuelles ultrasoniques sont déposées, dans une phase formant une acquisition de données, tout d'abord dans le système de traitement d'images (1) comme données brutes, respectivement, conjointement avec une en-tête d'image qui contient au moins les données de position et d'orientation, le système de traitement d'images (1) transformant le jeu d'images brutes (18) en jeux de données tridimensionnelles (19),

**caractérisé par** des moyens qui, lors de la transformation des données brutes (18) en jeu de données (19), pour chaque voxel (20), produisent la valeur d'image correspondante par formation d'une valeur de sommation pondérée ou d'une valeur moyenne pondérée, selon la formule :

$$GV = \{G1^*(C - X1) + G2^*(C - X2) + ..... + Gn^*(C-Xn)\} / \{(C - X1) + (C - X2) + ..... + (C - Xn)\}$$

où

GV est la valeur d'image du voxel respectif (20),

G1 ....... Gn sont les valeurs d'images des points d'images individuelles observables se trouvant le plus près du voxel respectif (20), et

X1, X2, ....... Xn la distance respective de ces points des images individuelles par rapport au voxel respectif (20), la distance respective ne dépassant pas une distance maximale C.

**7.** Dispositif selon la revendication 6, **caractérisé en ce que**, lors de la transformation, on choisit tout d'abord, selon un programme prédéterminé, le voxel respectif (20) ou sa position et, ensuite, on forme la valeur d'image du voxel (20).

**8.** Dispositif selon l'une quelconque des revendications 6 et 7, **caractérisé en ce que** le récepteur ou les bobines réceptrices de ce système capteur sont aménagés sur la tête à ultrasons (7) ou sur le support (17) fixé à la tête à ultrasons, et **en ce que** le système de saisie d'images (1) calibre les données de position et d'orientation délivrées par le système capteur en utilisant des paramètres qui font intervenir la référence spatiale entre le récepteur (11) du système capteur et son orientation dans l'espace et la position du plan image de la tête à ultrasons (7),

où, par exemple, le calibrage des données de position et d'orientation se fait dans une phase de post-traitement suivant la phase d'acquisition de données et/ou, par exemple, le calibrage des données de position et d'orientation se fait au cours de la phase d'acquisition de données et, de préférence, les données calibrées de position et d'orientation sont stockées dans l'en-tête d'image respective.

**9.** Dispositif selon l'une quelconque des revendications 6, 7 et 8, **caractérisé par** un appareil ECG raccordé au système de traitement d'images (1) pour produire un signal définissant un cycle ECG, en particulier un signal représentant les pointes R du cycle ECG, où le système de traitement d'images (1) présente des moyens pour stocker dans l'en-tête d'image une valeur qui représente la position de phase entre un moment défini du cycle ECG, par exemple les pointes R, et l'introduction d'une image individuelle ultrasonique sur le système de traitement d'images.

**10.** Dispositif selon l'une quelconque des revendications 6 à 9, **caractérisé en ce que**, pour générer un jeu de données statiques tridimensionnelles, l'introduction des images ultrasoniques sur le système de traitement d'images (1) se fait en synchronisme avec le cycle ECG, respectivement, dans une fenêtre temporelle prédéterminée de ce cycle, et/ou **en ce que**, pour générer un jeu (19, 19') de données dynamiques tridimensionnelles, l'introduction des images individuelles ultrasoniques (1) se fait en continu sur le système de traitement d'images (1) et/ou **en ce que** le système de traitement d'images présente des moyens pour stocker les valeurs d'images dans le jeu de données tridimensionnelles (19, 19'), respectivement, en fonction de la position de phase dans le cycle cardiaque, et/ou **en ce qu'**on utilise un appareil (13) pour saisir le cycle de respiration et **en ce que** l'on stocke dans l'en-tête d'image une valeur de mesure qui détermine la position de phase dans le cycle respiratoire.

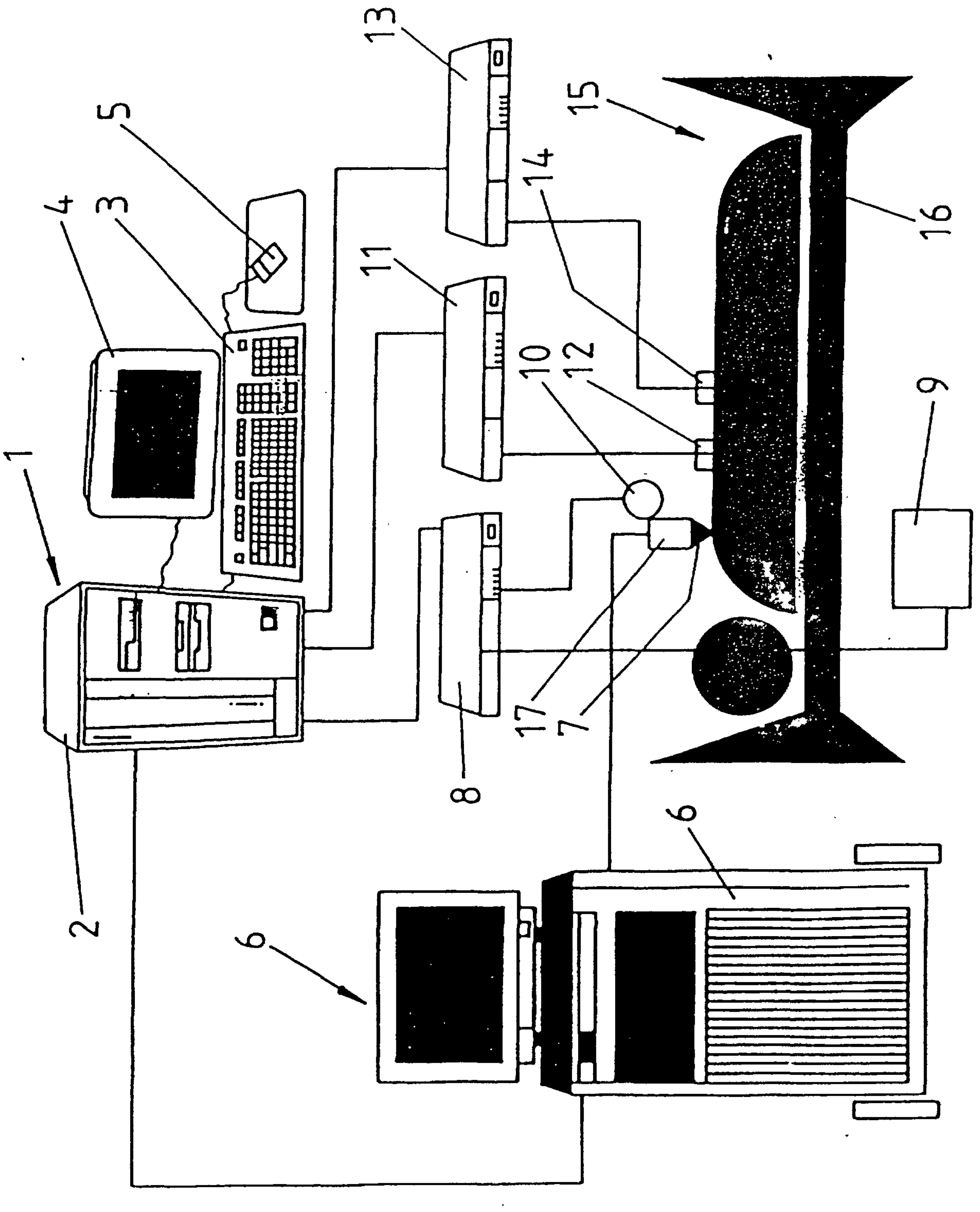


Fig. 1

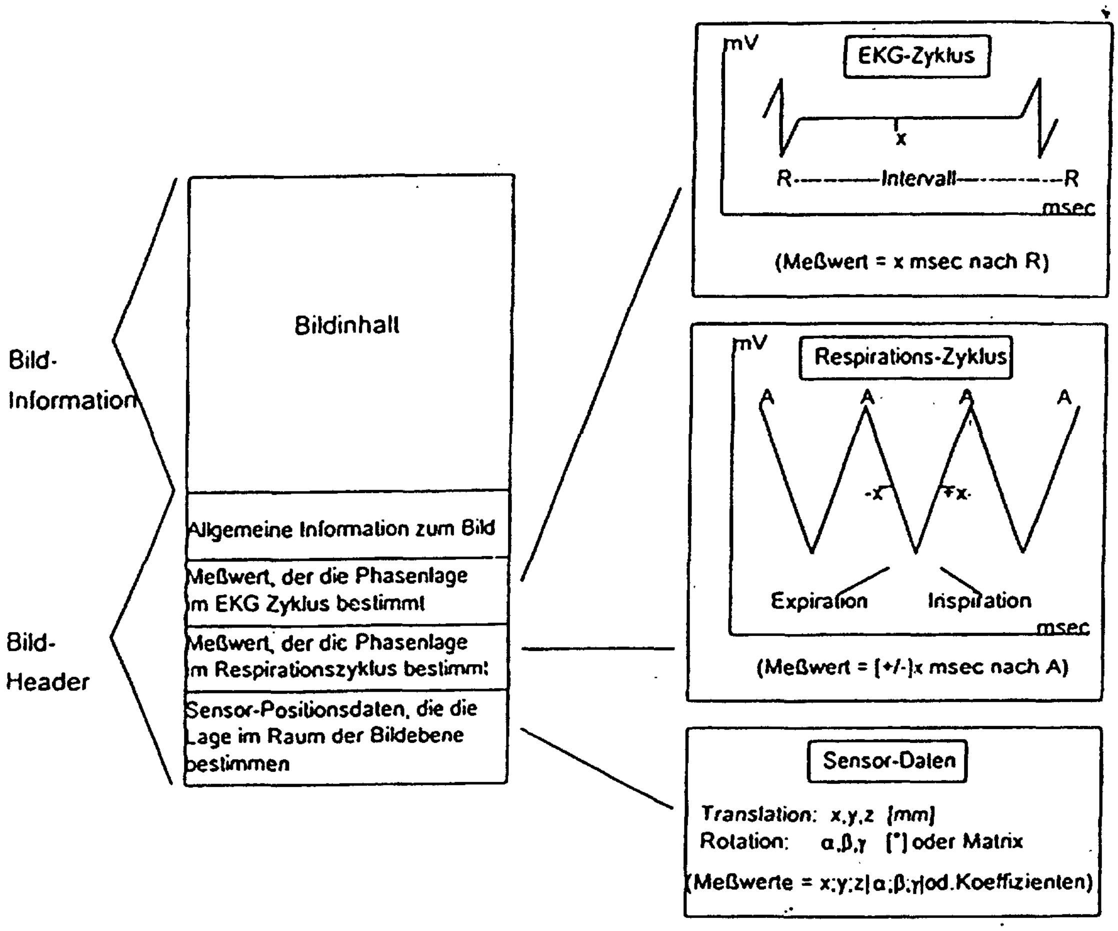
Bild-
Information
Bild-
Header
Bildinhalt
Allgemeine Information zum Bild
Meßwert, der die Phasenlage im EKG Zyklus bestimmt
Meßwert, der die Phasenlage im Respirationszyklus bestimmt
Sensor-Positionsdaten, die die Lage im Raum der Bildebene bestimmen
mV
EKG-Zyklus
x
R——Intervall——R
msec
(Meßwert = x msec nach R)
mV
Respirations-Zyklus
A
A
A
A
-x
+x
Expiration
Inspiration
msec
(Meßwert = (+/-)x msec nach A)
Sensor-Daten
Translation: x,y,z (mm)
Rotation: α,β,γ (°) oder Matrix
(Meßwerte = x;y;z|α;β;γ|od.Koeffizienten)

Fig 2

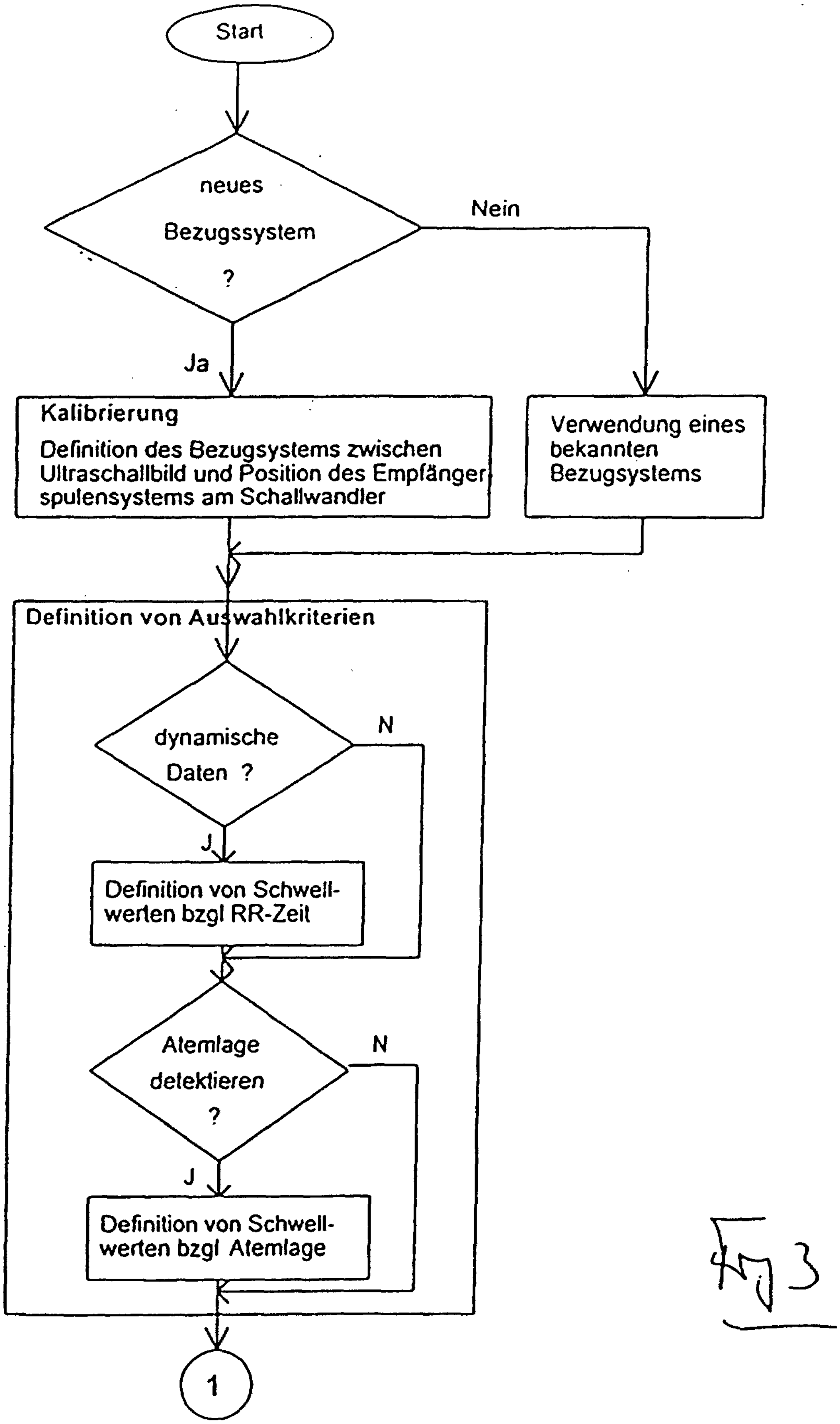
Start
neues
Bezugssystem
?
Nein
Ja
Kalibrierung
Definition des Bezugsystems zwischen Ultraschallbild und Position des Empfänger spulensystems am Schallwandler
Verwendung eines bekannten Bezugsystems
Definition von Auswahlkriterien
dynamische Daten ?
N
J
Definition von Schwellwerten bzgl RR-Zeit
Atemlage detektieren ?
N
J
Definition von Schwellwerten bzgl Atemlage
1

Fig 3

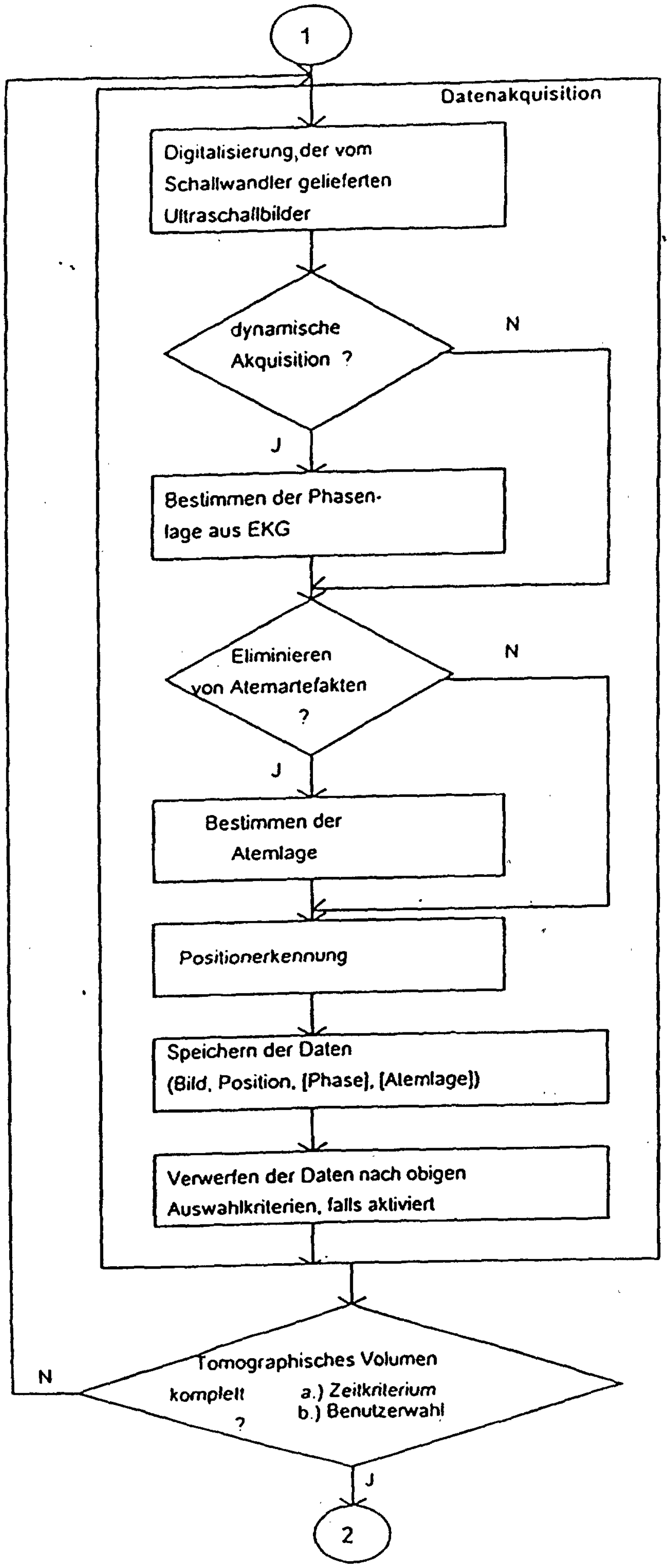
1
Datenakquisition
Digitalisierung,der vom Schallwandler gelieferten Ultraschallbilder
dynamische Akquisition ?
N
J
Bestimmen der Phasenlage aus EKG
Eliminieren von Atemartefakten ?
N
J
Bestimmen der Atemlage
Positionerkennung
Speichern der Daten (Bild, Position, [Phase], [Atemlage])
Verwerfen der Daten nach obigen Auswahlkriterien, falls aktiviert
Tomographisches Volumen komplett ?
a.) Zeitkriterium
b.) Benutzerwahl
N
J
2

Fig 4

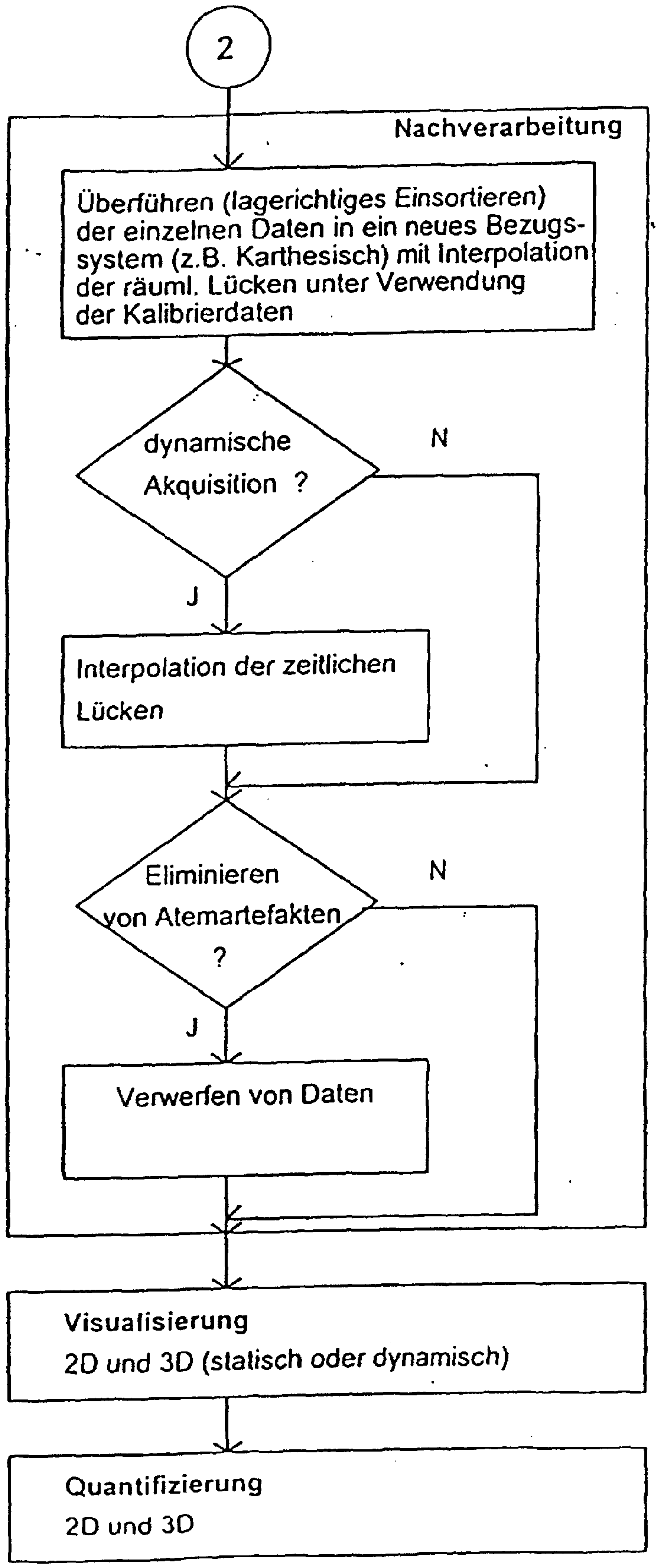
2
Nachverarbeitung
Überführen (lagerichtiges Einsortieren) der einzelnen Daten in ein neues Bezugssystem (z.B. Karthesisch) mit Interpolation der räuml. Lücken unter Verwendung der Kalibrierdaten
dynamische Akquisition ?
N
J
Interpolation der zeitlichen Lücken
Eliminieren von Atemartefakten ?
N
J
Verwerfen von Daten
Visualisierung
2D und 3D (statisch oder dynamisch)
Quantifizierung
2D und 3D

Fig 3

Fig. 6

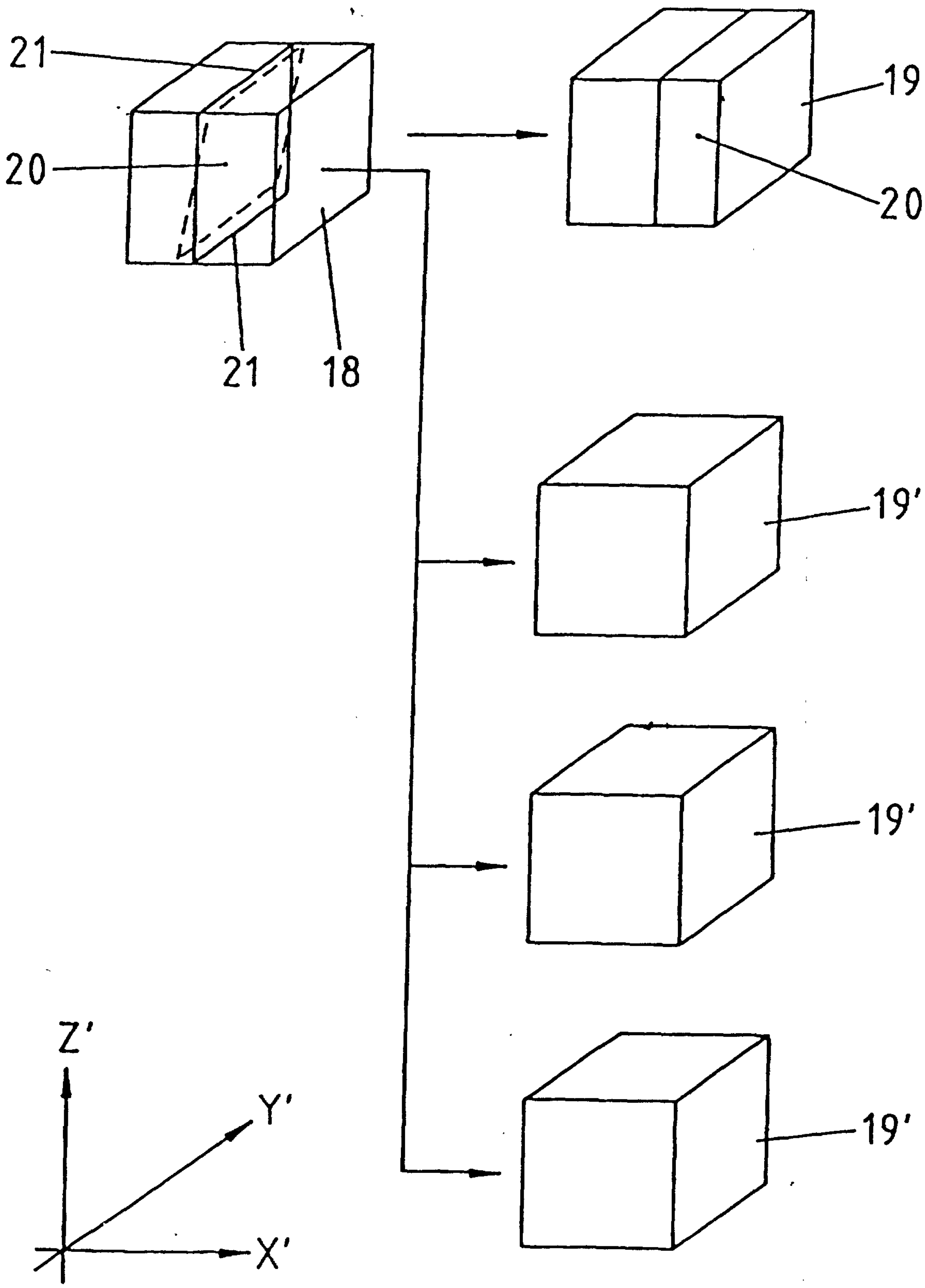

Fig. 7